# EUROPEAN PATENT SPECIFICATION

(11) **EP 1 810 628 B1**
(45) Date of publication and mention of the grant of the patent: **15.07.2009**
(21) Application number: 07001481.6
(22) Date of filing: 24.01.2007
(51) Int. Cl.: A61B 18/12, A61B 18/14

(54) **System for tissue sealing**
Anordnung zur Gewebeabdichtung
Système d'obturation des tissus

(30) Priority: 24.01.2006 US 338480
(43) Date of publication of application: 25.07.2007
(73) Proprietor: Covidien AG, 8212 Neuhausen am Rheinfall (CH)
(72) Inventor: Odom, Darren, Longmont, Colorado 80501 (US)
(74) Representative: HOFFMANN EITLE

(56) References cited:
- EP-A- 1 500 378
- EP-A- 1 535 581
- EP-A- 1 707 143
- US-A1- 2002 052 599

## Description

### BACKGROUND

### Technical Field

The present disclosure relates to an electrosurgical system and method for performing electrosurgical procedures. More particularly, the present disclosure relates to sealing vessels, wherein energy is administered at a constant predetermined voltage for a predetermined period of time.

### Background of Related Art

Electrosurgery involves application of high radio frequency electrical current to a surgical site to cut, ablate, or coagulate tissue. In monopolar electrosurgery, a source or active electrode delivers radio frequency energy from the electrosurgical generator to the tissue and a return electrode carries the current back to the generator. In monopolar electrosurgery, the source electrode is typically part of the surgical instrument held by the surgeon and applied to the tissue to be treated. A patient return electrode is placed remotely from the active electrode to carry the current back to the generator.

In bipolar electrosurgery, one of the electrodes of the hand-held instrument functions as the active electrode and the other as the return electrode. The return electrode is placed in close proximity to the active electrode such that an electrical circuit is formed between the two electrodes (e.g., electrosurgical forceps). In this manner, the applied electrical current is limited to the body tissue positioned between the electrodes. When the electrodes are sufficiently separated from one another, the electrical circuit is open and thus inadvertent contact of body tissue with either of the separated electrodes does not cause current to flow.

Bipolar electrosurgery generally involves the use of forceps. A forceps is a pliers-like instrument which relies on mechanical action between its jaws to grasp, clamp and constrict vessels or tissue. So-called "open forceps" are commonly used in open surgical procedures whereas "endoscopic forceps" or "laparoscopic forceps" are, as the name implies, used for less invasive endoscopic surgical procedures. Electrosurgical forceps (open or endoscopic) utilize mechanical clamping action and electrical energy to effect hemostasis on the clamped tissue. The forceps include electrosurgical conductive plates which apply the electrosurgical energy to the clamped tissue. By controlling the intensity, frequency and duration of the electrosurgical energy applied through the conductive plates to the tissue, the surgeon can coagulate, cauterize and/or seal tissue.

Tissue or vessel sealing is a process of liquefying the collagen, elastin and ground substances in the tissue so that they reform into a fused mass with significantly-reduced demarcation between the opposing tissue structures. Cauterization involves the use of heat to destroy tissue and coagulation is a process of desiccating tissue wherein the tissue cells are ruptured and dried.

Since tissue sealing procedures involve more than simply cauterizing tissue, to create an effective seal the procedures involve precise control of a variety of factors. In order to affect a proper seal in vessels or tissue, it has been determined that two predominant mechanical parameters must be accurately controlled: the pressure applied to the tissue; and the gap distance between the electrodes (i.e., distance between opposing jaw members when or opposing electrically conductive plates closed about tissue).

Many of the instruments of the past include blade members or shearing members which simply cut tissue in a mechanical and/or electromechanical manner. Other instruments generally rely on clamping pressure alone to procure proper sealing thickness and are often not designed to take into account gap tolerances and/or parallelism and flatness requirements which are parameters which, if properly controlled, can assure a consistent and effective tissue seal.

A continual need exists to develop new electrosurgical systems and methods which allow for creation of durable vessel seals capable of withstanding higher burst pressures.

EP 1 500 378 discloses a system for electrosurgically sealing a tissue.

### SUMMARY

The present disclosure relates to a vessel or tissue sealing system and method. In particular, the system discloses a bipolar forceps having two jaw members configured for grasping tissue. Each of the jaw members include a sealing plate which communicates electrosurgical energy to the tissue. At the start of the procedure, the system transmits an initial interrogatory pulse for determining initial tissue impedance. Based on the initial impedance the system determines the optimum pressure, voltage, and duration of energy application. During the procedure constant pressure is applied to tissue and electrosurgical energy is applied at constant voltage for the predetermined duration.

One embodiment according to the present invention, and as recited in claim 1, relates to an electrosurgical bipolar forceps for sealing tissue. The forceps includes one or more shaft members having an end effector assembly disposed at the distal end. The end effector assembly includes two jaw members movable from a first position to a closed position wherein the jaw members cooperate to grasp tissue at constant pressure. Each of the jaw members includes an electrically conductive sealing plate connected to a first energy source which communicates electrosurgical energy through the tissue held therebetween. The electrosurgical energy is communicated at constant voltage. The electrically conductive sealing plates are operably connected to sensor circuitry which is configured to measure initial tissue impedance and transmit an initial impedance value to a controller. The controller determines the constant pressure and the constant voltage to be applied to the tissue based on the initial impedance value.

Another preferred embodiment of the present invention, and as recited in claim 8, is directed to an electrosurgical system. The system includes an electrosurgical generator for supplying electrosurgical energy and the above described bipolar forceps for treating tissue.

Also disclosed, but not recited in the claims, is a method for sealing tissue. The method includes the steps of providing an electrosurgical bipolar forceps which includes one or more shaft members having an end effector assembly disposed at the distal end. The end effector assembly includes two jaw members movable from a first open position to a closed position wherein the jaw members cooperate to grasp tissue at constant pressure. Each of the jaw members includes an electrically conductive sealing plate connected to an energy source which communicates electrosurgical energy through the tissue held therebetween. The electrosurgical energy is communicated at constant voltage. The method also includes the steps of measuring initial tissue impedance and transmitting an initial impedance value to a controller and determining the constant pressure and the constant voltage to be applied to the tissue based on the initial impedance value.

### BRIEF DESCRIPTION OF THE DRAWINGS

Various embodiments of the present disclosure are described herein with reference to the drawings wherein:
Fig. 1 is a perspective view of one embodiment of an electrosurgical system according to the present disclosure;
Fig. 2 is a schematic block diagram of a generator according to the present disclosure;
Fig. 3 is a rear, perspective view of the end effector of Fig. 1 shown with tissue grasped therein;
Fig. 4 is a side, partial internal view of an endoscopic forceps according to the present disclosure;
Fig. 5 shows a flow chart showing a sealing method using the endoscopic bipolar forceps according to the present disclosure;
Fig. 6 shows a graph illustrating the changes occurring in tissue impedance during sealing utilizing the method shown in Fig. 5; and
Fig. 7 is a perspective view of an open bipolar forceps according to the present disclosure.

### DETAILED DESCRIPTION

Particular embodiments of the present disclosure will be described hereinbelow with reference to the accompanying drawings. In the following description, well-known functions or constructions are not described in detail to avoid obscuring the present disclosure in unnecessary detail. Those skilled in the art will understand that the invention according to the present disclosure may be adapted for use with either an endoscopic instrument or an open instrument. It should also be appreciated that different electrical and mechanical connections and other considerations apply to each particular type of instrument, however, the novel aspects with respect to vessel sealing are generally consistent with respect to both the open or endoscopic designs.

In the drawings and in the description which follows, the term "proximal", refers to the end of the forceps 10 which is closer to the user, while the term "distal" refers to the end of the forceps which is further from the user.

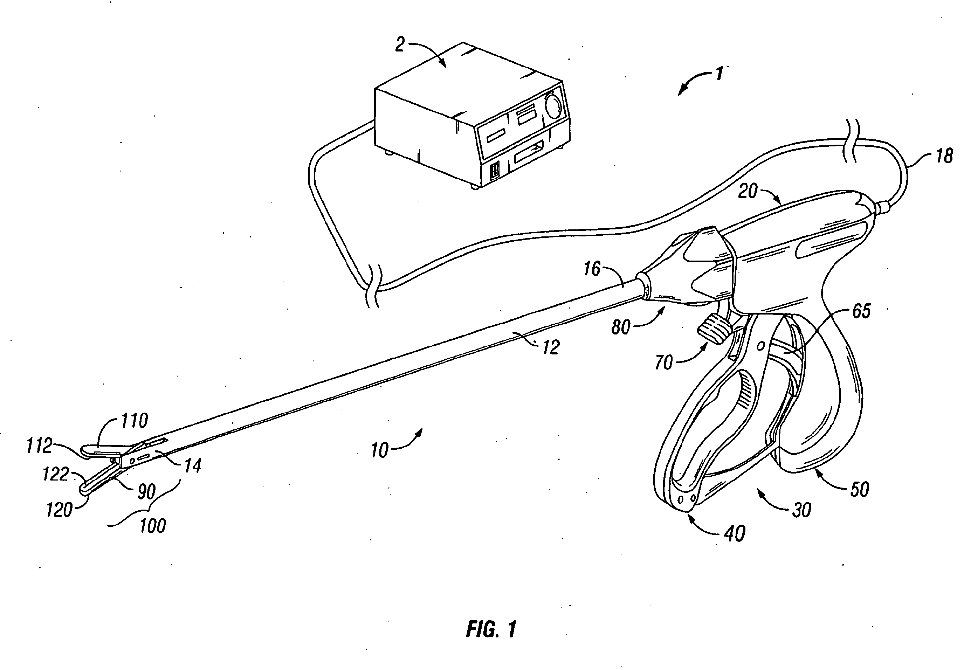

Fig. 1 is a schematic illustration of an electrosurgical system 1. The system 1 includes an electrosurgical forceps 10 for treating tissue of a patient. Electrosurgical RF energy is supplied to the forceps 10 by a generator 2 via a cable 18 allowing the forceps to seal tissue.

As shown in Fig. 1, the forceps 10 is an endoscopic vessel sealing bipolar forceps. The forceps 10 is configured to support an effector assembly 100. More particularly, forceps 10 generally includes a housing 20, a handle assembly 30, a rotating assembly 80, and a trigger assembly 70 which mutually cooperate with the end effector assembly 100 to grasp, seal and, if required, divide tissue. The forceps 10 also includes a shaft 12 which has a distal end 14 which mechanically engages the end effector assembly 100 and a proximal end 16 which mechanically engages the housing 20 proximate the rotating assembly 80.

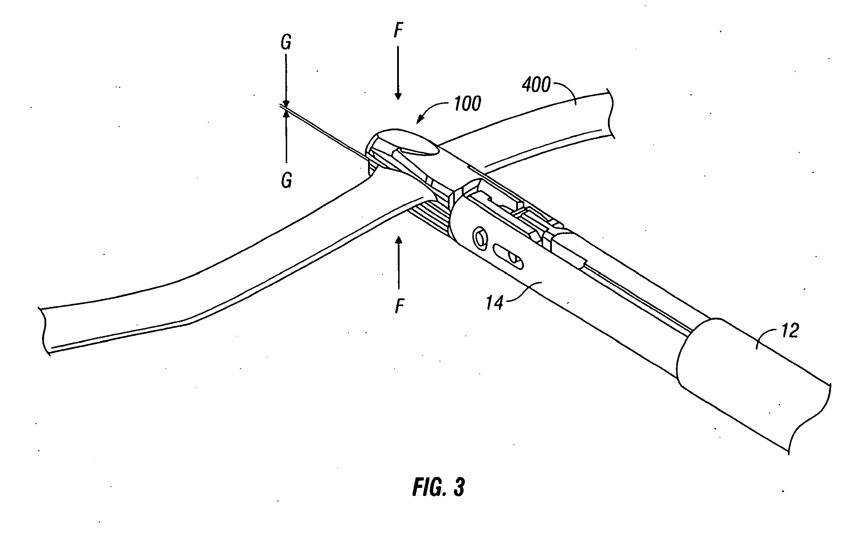

The forceps 10 also includes a plug (not shown) which connects the forceps 10 to a source of electrosurgical energy, e.g., the generator 2, via cable 18. Handle assembly 30 includes a fixed handle 50 and a movable handle 40. Handle 40 moves relative to the fixed handle 50 to actuate the end effector assembly 100 and enable a user to grasp and manipulate tissue 400 as shown in Fig. 3.

Referring to Figs. 1, 3 and 4, the end effector assembly 100 includes a pair of opposing jaw members 110 and 120 each having an electrically conductive sealing plate 112 and 122, respectively, attached thereto for conducting electrosurgical energy through tissue 400 held therebetween. More particularly, the jaw members 110 and 120 move in response to movement of the handle 40 from an open position to a closed position. In open position the sealing plates 112 and 122 are disposed in spaced relation relative to one another. In a clamping or closed position the sealing plates 112 and 122 cooperate to grasp tissue and apply electrosurgical energy thereto.

The jaw members 110 and 120 are activated using a drive assembly (not shown) enclosed within the housing 20. The drive assembly cooperates with the movable handle 40 to impart movement of the jaw members 110 and 120 from the open position to the clamping or closed position. Examples of a handle assemblies are shown and described in commonly-owned U.S. Patent Application US 2003-0229344 A1 entitled "VESSEL SEALER AND DIVIDER AND METHOD MANUFACTURING SAME" and commonly owned U.S. Patent US 7,156,846 entitled "VESSEL SEALER AND DIVIDER FOR USE WITH SMALL TROCARS AND CANNULAS".

Jaw members 110 and 120 also include outer housings 116 and 126 which together with the dimensions of the conductive plates 112 and 122 of the jaw members 110 and 120 are configured to limit and/or reduce many of the known undesirable effects related to tissue sealing, e.g., flashover, thermal spread and stray current dissipation.

The handle assembly 30 of this particular disclosure may include a four-bar mechanical linkage which provides a unique mechanical advantage when sealing tissue between the jaw members 110 and 120. Once the desired position for the sealing site is determined and the jaw members 110 and 120 are properly positioned, handle 40 may be compressed fully to lock the electrically conductive sealing platens 112 and 122 in a closed position against the tissue. The details relating to the inter-cooperative relationships of the inner-working components of one envisioned forceps 10 are disclosed in the above-cited commonly-owned U.S. Patent Application US 2003-0229344 A1. Another example of an endoscopic handle assembly which discloses an off-axis, lever-like handle assembly, is disclosed in the above-cited U.S. Patent US 7,156,846.

The forceps 10 also includes a rotating assembly 80 mechanically associated with the shaft 12 and the drive assembly (not shown). Movement of the rotating assembly 80 imparts similar rotational movement to the shaft 12 which, in turn, rotates the end effector assembly 100. Various features along with various electrical configurations for the transference of electrosurgical energy through the handle assembly 20 and the rotating assembly 80 are described in more detail in the above-mentioned commonly-owned U.S. Patent Application US 2003-0229344 A1 and Patent US 7,156,846.

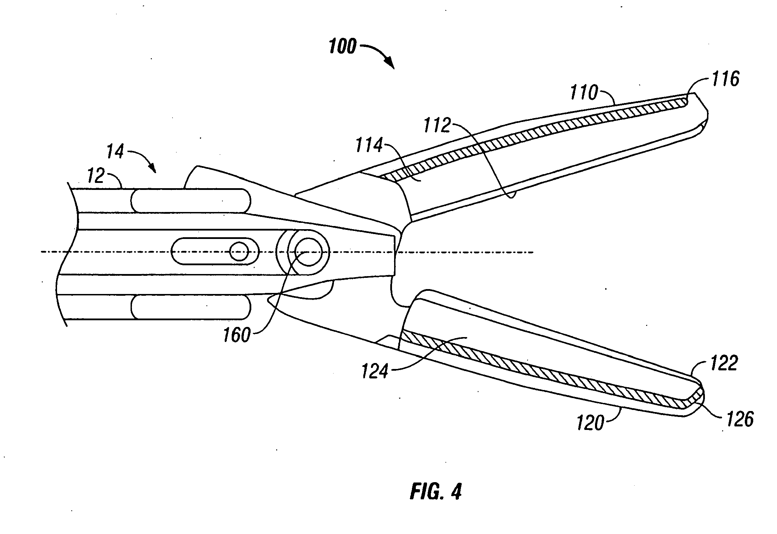

As best seen with respect to Figs. 1 and 4, the end effector assembly 100 attaches to the distal end 14 of shaft 12. The jaw members 110 and 120 are pivotable about a pivot 160 from the open to closed positions upon relative reciprocation, i.e., longitudinal movement, of the drive assembly (not shown). Again, mechanical and cooperative relationships with respect to the various moving elements of the end effector assembly 100 are further described by example with respect to the above-mentioned commonly-owned U.S. Patent Application US 2003-0229344 A1 and Patent US.7,156,846.

It is envisioned that the forceps 10 may be designed such that it is fully or partially disposable depending upon a particular purpose or to achieve a particular result. For example, end effector assembly 100 may be selectively and releasably engageable with the distal end 14 of the shaft 12 and/or the proximal end 16 of the shaft 12 may be selectively and releasably engageable with the housing 20 and handle assembly 30. In either of these two instances, the forceps 10 may be either partially disposable or reposable, such as where a new or different end effector assembly 100 or end effector assembly 100 and shaft 12 are used to selectively replace the old end effector assembly 100 as needed.

The generator 2 includes input controls (e.g., buttons, activators, switches, touch screen, etc.) for controlling the generator 2. In addition, the generator 2 includes one or more display screens for providing the surgeon with variety of output information (e.g., intensity settings, treatment complete indicators, etc.). The controls allow the surgeon to adjust power of the RF energy, waveform, and other parameters to achieve the desired waveform suitable for a particular task (e.g., coagulating, tissue sealing, intensity setting, etc.). It is also envisioned that the forceps 10 may include a plurality of input controls which may be redundant with certain input controls of the generator 2. Placing the input controls at the forceps 10 allows for easier and faster modification of RF energy parameters during the surgical procedure without requiring user interaction at the generator 2.

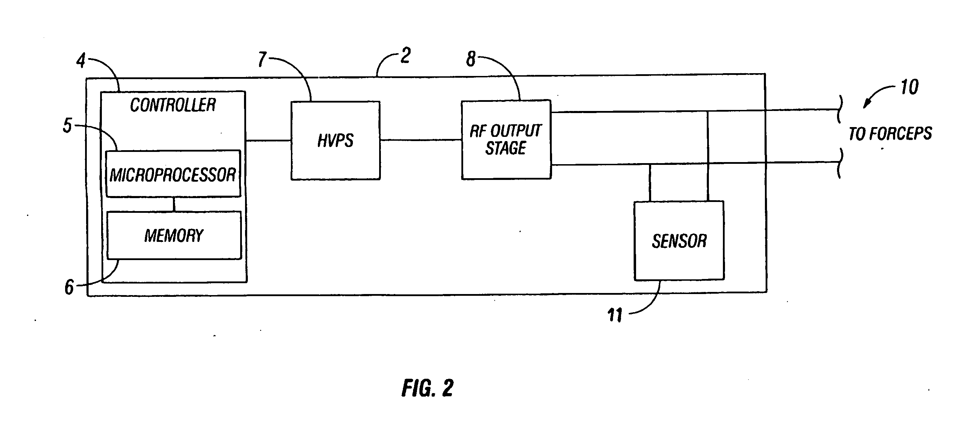

Fig. 2 shows a schematic block diagram of the generator 2 having a controller 4, a high voltage DC power supply 7 ("HVPS"), an RF output stage 8, and a sensor circuitry 11. The DC power supply 7 provides DC power to an RF output stage 8 which then converts DC power into RF energy and delivers the RF energy to the forceps 10. The controller 4 includes a microprocessor 5 operably connected to a memory 6 which may be volatile type memory (e.g., RAM) and/or non-volitile type memory (e.g., flash media, disk media, etc.). The microprocessor 5 includes an output port which is operably connected to the HVPS 7 and/or RF output stage 8 allowing the microprocessor 5 to control the output of the generator 2 according to either open and/or closed control loop schemes. A closed loop control scheme may be a feedback control loop wherein the sensor circuitry 11, which may include a plurality of sensing mechanisms (e.g., tissue impedance, tissue temperature, output current and/or voltage, etc.), provides feedback to the controller 4. The controller 4 then signals the HVPS 7 and/or RF output stage 8 which then adjust DC and/or RF power supply, respectively. The controller 4 also receives input signals from the input controls of the generator 2 and/or forceps 10. The controller 4 utilizes the input signals to adjust power supplied by the generator 2 and/or performs other control functions thereon.

With respect to this particular embodiment, it is known that sealing of the tissue 400 is accomplished by virtue of a unique combination of gap control, pressure and electrical control. In other words, controlling the intensity, frequency and duration of the electrosurgical energy applied to the tissue through the sealing plate 112 and 122 are important electrical considerations for sealing tissue. In addition, two mechanical factors play an important role in determining the resulting thickness of the sealed tissue and the effectiveness of the seal, i.e., the pressure applied between the opposing jaw members 110 and 120 (between about 3 kg/cm2 to about 16kg/cm2) and the gap distance "G" between the opposing sealing plates 112 and 122 of the jaw members 110 and 120, respectively, during the sealing process (between about 0.001 inches to 0.006 inches). One or more stop members 90 are typically employed on one or both sealing plates to control the gap distance. A third mechanical factor has recently been determined to contribute to the quality and consistency of a tissue seal, namely the closure rate of the electrically conductive surfaces or sealing plates during activation.

Since the forceps 10 applies energy through electrodes, each of the jaw members 110 and 120 includes a pair of electrically sealing plates 112, 122 respectively, disposed on an inner-facing surface thereof. Thus, once the jaw members 110 and 120 are fully compressed about the tissue 400, the forceps 10 is now ready for selective application of electrosurgical energy as shown in Fig. 4. At that point, the electrically sealing plates 112 and 122 cooperate to seal tissue 400 held therebetween upon the application of electrosurgical energy.

The system 1 according to present disclosure regulates application of energy and pressure to achieve an effective seal capable of withstanding high burst pressures. The generator 2 applies energy to tissue at constant voltage and regulated the pressure. Pressure is regulated by closing jaw members 110 and 120 at a predetermined rate. Energy application is regulated by the controller 4 pursuant to an algorithm stored within the memory 6. The algorithm maintains energy supplied to the tissue at constant voltage. The algorithm varies output based on the type of tissue being sealed. For instance, thicker tissue requires more power applied thereto, whereas thinner tissue requires less. Therefore the algorithm adjusts the output based on tissue type by modifying specific variables (e.g., voltage being maintained, duration of power application etc.).

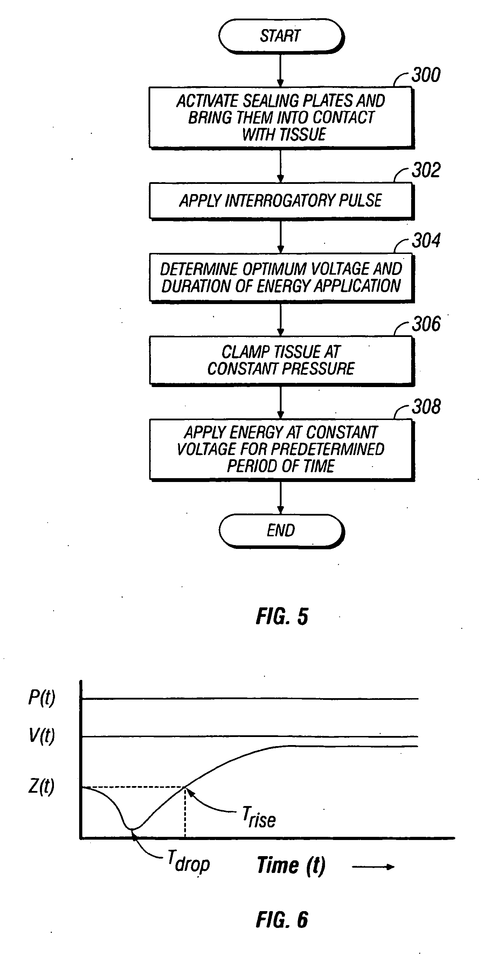

The algorithm will be discussed in further detail below with reference to Fig. 5. In addition, Fig. 6 shows a graph illustrating the changes that are contemplated to occur to collagen when it is subjected to sealing using the method of Fig. 5.

During step 300, the sealing plates 112 and 122 are activated and are in contact with the tissue 400 but are not fully closed. When the sealing plates 112 and 122 contact the tissue 400, in step 302, an interrogatory pulse is applied to the tissue 400. The interrogatory pulse is used to sense initial tissue impedance via the sensor circuitry 11. The pulse is of small voltage and of short duration.

In step 304, the initial impedance is transmitted to the controller 4 which determines optimum voltage for the sealing procedure and the duration of energy application. In particular, the microprocessor 5 may use a look-up table located in the memory 6. The look-up table may have voltage and duration values for a plurality of initially impedance ranges. For instance, if the initial impedance is measured to be from about 70 to about 100 Ohms, for a particular range the look-up table provides the optimum voltage value of 150 V and duration of 30 sec. The microprocessor 5 extracts the values from the look-up table and regulates the generator 2 accordingly.

It is also envisioned that the optimum voltage and duration of the energy application may be set manually. The initial impedance may be displayed on a display screen of the generator 2 and the surgeon may set the optimum voltage and duration according to the measured initial impedance.

In step 305, the forceps 10 grasps and begins to apply pressure to the tissue 400 using the jaw members 110 and 120. Pressure being applied is held constant for the duration of the sealing procedure as shown by the line P(t).

Various methods and devices are contemplated to automatically regulate the closure of the jaw members 110 and 120 about tissue to keep the pressure constant during the sealing process. For example, the forceps 10 may be configured to include a ratchet mechanism which initially locks the jaw members 110 and 120 against the tissue under a desired tissue pressure and then increases the pressure according to the command from the microprocessor 5 to an optimum tissue pressure. The ratchet mechanism is configured to adjust the pressure based on the tissue reaction. It is also envisioned that the pressure may be controlled in a similar manner towards the end of the seal cycle, i.e., release pressure. A similar or the same ratchet mechanism may be employed for this purpose as well.

Other controllable closure mechanisms are also envisioned which may be associated with the handle assembly 30, the housing 20 and/or the jaw members 110 and 120 (i.e., gearing mechanisms, pressure-assist mechanisms, hydraulic mechanisms, electro-mechanical mechanisms, etc.). Any of these mechanisms may be housed in the housing 20 or form a part of each particular structure.

It is also envisioned that one or more stop members 90 may be selectively controllable to regulate the closure pressure and gap distance to affect the seal. Commonly-owned U.S. Patent Application US 2005-0021027 A1 describes one such variable stop system which may be used for this purpose.

In step 308, electrosurgical energy is applied to tissue at constant voltage. The collagen contained therein is denatured and becomes more mobile (i.e., liquefies). Simultaneously, the water contained within the tissue 400 is allowed to escape from the sealing site. As a result, the peak temperature at which a seal is created is reduced. Thereafter, the previously melted collagen is mixed in order to allow for its structural components (e.g., polymers) to intertwine. Mixing can be achieved by applying electrosurgical energy of predetermined frequency to the sealing site through the sealing plates 112 and 122 under a predetermined pressure. The optimum frequency and amplitude of the waves depends on the collagen structures which are being mixed and may be automatically controlled as specified above. Once the collagen is mixed, it is further cured by continual application of electrosurgical energy and pressure.

Energy application may be terminated when the predetermined duration period has expired. It is envisioned that energy application may stop once a predetermined amount of energy has been applied to the tissue. Thus, the same or a different look-up table may also store total energy to be applied to tissue to create a seal. After initial impedance is obtained the microprocessor 5 loads the total energy value and adjusts the output of the generator accordingly.

Duration of energy application may be iteratively determined during the procedure. The microprocessor 5 includes a clock which allows the microprocessor 5 to determine the duration of energy application during the sealing process. It is further envisioned that the controller 4 may calculate the amount of time it takes for the initial impedance to drop and/or the time for the impedance to rise back to the original value, both values are shown as T_{droP} and Tᵣᵢₛₑ on the graph of Fig. 5.

The algorithm according to the present disclosure allows for slow desiccation of tissue and for collagen to denature slowly as well. Application of energy for a relatively long period of time (e.g., 30 seconds) at constant voltage allows tissue to change very slowly. As desiccation progresses, the resulting seal gains plastic-like qualities, becoming hard and clear, which makes the seal capable of withstanding higher burst pressures.

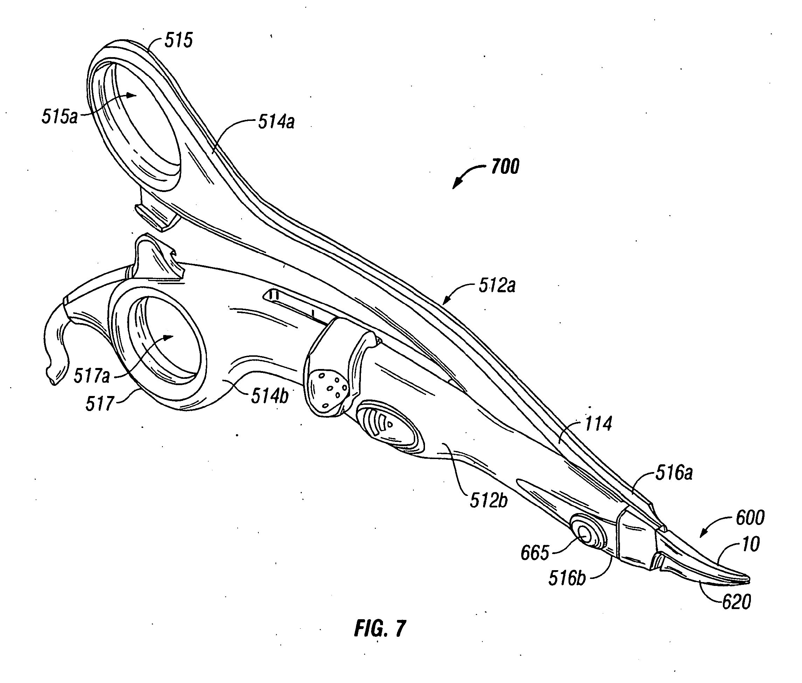

From the foregoing and with reference to the various figure drawings, those skilled in the art will appreciate that certain modifications can also be made to the present disclosure without departing from the scope of the same. For example and as mentioned above, it is contemplated that any of the various jaw arrangements disclosed herein may be employed on an open forceps such as the open forceps 700 shown in Fig. 7. The forceps 700 includes an end effector assembly 600 which is attached to the distal ends 516a and 516b of shafts 512a and 512b, respectively. The end effector assembly 600 includes pair of opposing jaw members 610 and 620 which are pivotally connected about a pivot pin 665 and which are movable relative to one another to grasp vessels and/or tissue. Each of the opposing jaw members 610, 620 includes electrically sealing plates 112, 122 allowing the open forceps 700 to be used for clamping tissue for sealing, coagulation or cauterization.

Each shaft 512a and 512b includes a handle 515 and 517, respectively, disposed at the proximal end 514a and 514b thereof which each define a finger hole 515a and 517a, respectively, therethrough for receiving a finger of the user. Finger holes 515a and 517a facilitate movement of the shafts 512a and 512b relative to one another which, in turn, pivot the jaw members 610 and 620 from an open position wherein the jaw members 610 and 620 are disposed in spaced relation relative to one another to a clamping or closed position wherein the jaw members 610 and 620 cooperate to grasp tissue or vessels therebetween. Further details relating to one particular open forceps are disclosed in commonly-owned U.S. Patent Application US 2005-0154387 A1 filed October 8, 2004 entitled "OPEN VESSEL SEALING INSTRUMENT WITH CUTTING MECHANISM AND DISTAL LOCKOUT".

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. An electrosurgical bipolar forceps (10) for sealing tissue (400), comprising:
at least one shaft member (12) having an end effector assembly (100) disposed at a distal end (14) thereof, the end effector assembly including two jaw members (110, 120) movable from a first position in spaced relation relative to one another to at least one subsequent position wherein the jaw members cooperate to grasp tissue therebetween at constant pressure;
each of the jaw members including an electrically conductive sealing plate (112, 122) adapted to connect to an energy source (2) which communicates electrosurgical energy through tissue held therebetween, wherein the electrosurgical energy is communicated at constant voltage; and
sensor circuitry (11) operably connected to the electrically conductive sealing plates, the sensor circuitry configured to measure initial tissue impedance and transmit an initial impedance value to a controller (4), **characterised by** the controller being configured to determine the constant pressure and the constant voltage to be applied to the tissue based on the initial impedance value.

2. An electrosurgical bipolar forceps according to claim 1, wherein electrosurgical energy is communicated for a predetermined duration.

3. An electrosurgical bipolar forceps according to claim 1 or 2, wherein the controller is further configured to determine the duration of a seal cycle based on the initial impedance value.

4. An electrosurgical bipolar forceps according to claim 1, 2 or 3, wherein the controller accesses a look-up table storing at least one of a duration value, a constant pressure value and a constant voltage value, the controller selects at least one of the duration value, the constant pressure value and the constant voltage value based on the initial impedance value.

5. An electrosurgical bipolar forceps according to any one of the preceding claims, further comprising:
a rotating assembly (80) mechanically associated with the shaft member, wherein rotation of the rotating assembly imparts similar rotational movement to the shaft member and the end effector assembly.

6. An electrosurgical bipolar forceps according to any one of the preceding claims, wherein electrosurgical energy is communicated until a predetermined amount of energy is supplied to tissue.

7. An electrosurgical bipolar forceps according to claim 6, wherein the controller determines the predetermined amount of energy based on the initial impedance value.

8. An electrosurgical system, comprising:
an electrosurgical generator (2) which supplies electrosurgical energy; and
bipolar forceps according to any one of the preceding claims, wherein each electrically conductive sealing plate is adapted to connect to the electrosurgical generator as the energy source.

## Patentansprüche

1. Elektrochirurgischer bipolarer Forceps (10) zum Versiegeln von Gewebe (400), umfassend:
zumindest ein Schaftelement (12) mit einer Endeffektoranordnung (100), die an einem distalen Ende (14) davon angeordnet ist, wobei die Endeffektoranordnung zwei Klauenelemente (110, 120) enthält, die von einer ersten Position in einer relativ zu einander beabstandeten Beziehung zu zumindest einer nachfolgenden Position, in der die Klauenelemente zusammenwirken, um Gewebe dazwischen mit einem konstanten Druck zu greifen, bewegbar sind;
wobei jedes der Klauenelemente eine elektrisch leitende Versiegelungsplatte (112, 122) enthält, die angepasst ist, um mit einer Energiequelle (2) verbunden zu sein, die elektrochirurgische Energie durch dazwischen gehaltenes Gewebe leitet, wobei die elektrochirurgische Energie mit einer konstanten Spannung geleitet wird; und
einen Sensorschaltkreis (11), der mit den elektrisch leitenden Versiegelungsplatten wirkverbunden ist, wobei der Sensorschaltkreis eingerichtet ist, um anfängliche Gewebeimpedanz zu messen und einen anfänglichen Impedanzwert an eine Steuerung (4) zu übertragen, **dadurch gekennzeichnet, dass** die Steuerung eingerichtet ist, um den konstanten Druck und die konstante Spannung, die an das Gewebe anzulegen sind, auf der Grundlage des anfänglichen Impedanzwerts zu bestimmen.

2. Elektrochirurgischer bipolarer Forceps nach Anspruch 1, wobei elektrochirurgische Energie über eine vorbestimmte Dauer geleitet wird.

3. Elektrochirurgischer bipolarer Forceps nach Anspruch 1 oder 2, wobei die Steuerung ferner eingerichtet ist, um die Dauer eines Versiegelungszyklus auf der Grundlage des anfänglichen Impedanzwerts zu bestimmen.

4. Elektrochirurgischer bipolarer Forceps nach Anspruch 1, 2 oder 3, wobei die Steuerung auf eine Nachschlagetabelle zugreift, die zumindest einen eines Dauerwerts, eines Werts eines konstanten Drucks und eines Werts einer konstanten Spannung speichert, wobei die Steuerung zumindest einen des Dauerwerts, des Werts des konstanten Drucks und des Werts der konstanten Spannung auf der Grundlage des anfänglichen Impedanzwerts auswählt.

5. Elektrochirurgischer bipolarer Forceps nach einem der vorstehenden Ansprüche, ferner umfassend:
eine sich drehende Anordnung (80), die mechanisch mit dem Schaftelement verbunden ist, wobei eine Drehung der sich drehenden Anordnung eine ähnliche Drehbewegung auf das Schaftelement und die Endeffektoranordnung überträgt.

6. Elektrochirurgischer bipolarer Forceps nach einem der vorstehenden Ansprüche, wobei elektrochirurgische Energie geleitet wird, bis eine vorbestimmte Menge von Energie einem Gewebe zugeführt ist.

7. Elektrochirurgischer bipolarer Forceps nach Anspruch 6, wobei die Steuerung die vorbestimmte Menge von Energie auf der Grundlage des anfänglichen Impedanzwerts bestimmt.

8. Elektrochirurgisches System, umfassend:
einen elektrochirurgischen Generator (2), der elektrochirurgische Energie zuführt; und
einen bipolaren Forceps nach einem der vorstehenden Ansprüche, wobei jede elektrisch leitende Versiegelungsplatte angepasst ist, um mit dem elektrochirurgischen Generator als die Energiequelle verbunden zu sein.

## Revendications

1. Forceps bipolaire électrochirurgical (10) pour l'obturation de tissu (400), comprenant:
au moins un élément d'arbre (12) ayant un ensemble formant organe terminal effecteur (100) disposé à une extrémité distale (14) de celui-ci, l'ensemble formant organe terminal effecteur incluant deux éléments de mâchoire (110, 120) déplaçables d'une première position, en une relation espacée l'un de l'autre, à au moins une position suivante dans laquelle les éléments de mâchoire coopèrent pour saisir le tissu entre eux à une pression constante;
chacun des éléments de mâchoire incluant une plaque de scellement électriquement conductrice (112, 122) apte à être connectée à une source d'énergie (2) qui communique de l'énergie électrochirurgicale à travers le tissu tenu entre eux, où l'énergie électrochirurgicale est fournie à une tension constante; et
des circuits de détection (11) fonctionnellement reliés aux plaques de scellement électriquement condutrices, les circuits de détection étant configurés pour mesurer l'impédance initiale du tissu et pour transmettre une valeur d'impédance initiale à un dispositif de commande (4), **caractérisé en ce que** le dispositif de commande est configuré pour déterminer la pression constante et la tension constante à appliquer au tissu sur la base de la valeur d'impédance initiale.

2. Forceps bipolaire électrochirurgical selon la revendication 1, dans lequel l'énergie électrochirurgicale est fournie pendant une durée prédéterminée.

3. Forceps bipolaire électrochirurgical selon la revendication 1 ou 2, où le dispositif de commande est configuré en outre pour déterminer la durée d'un cycle de scellement sur la base de la valeur d'impédance initiale.

4. Forceps bipolaire électrochirurgical selon la revendication 1, 2 ou 3, dans lequel le dispositif de commande accède à une table de consultation stockant au moins une parmi une valeur de durée, une valeur de pression constante et une valeur de tension constante, le dispositif de commande sélectionne au moins une parmi la valeur de durée, la valeur de pression constante et la valeur de tension constante sur la base de la valeur d'impédance initiale.

5. Forceps bipolaire électrochirurgical selon l'une quelconque des revendications précédentes, comprenant en outre:
un ensemble de rotation (80) mécaniquement associé à l'élément d'arbre, où la rotation de l'ensemble de rotation impartit un mouvement de rotation similaire à l'élément d'arbre et à l'ensemble formant organe terminal effecteur.

6. Forceps bipolaire électrochirurgical selon l'une quelconque des revendications précédentes, dans lequel l'énergie électrochirurgicale est communiquée jusqu'à ce qu'une quantité d'énergie prédéterminée ait été fournie au tissu.

7. Forceps bipolaire électrochirurgical selon la revendication 6, où le dispositif de commande détermine la quantité d'énergie prédéterminée basée sur la valeur d'impédance initiale.

8. Système électrochirurgical, comprenant:
un générateur électrochirurgical (2) qui fournit de l'énergie électrochirurgicale; et
un forceps bipolaire selon l'une quelconque des revendications précédentes, où chaque plaque de scellement électriquement conductrice est apte à être connectée au générateur électrochirurgical en tant que source d'énergie.
